(19) Europäisches Patentamt
European Patent Office
Office européen des brevets

(11) Veröffentlichungsnummer : **0 065 705**
**B1**

(12) # EUROPÄISCHE PATENTSCHRIFT

(45) Veröffentlichungstag der Patentschrift : **02.01.86**

(51) Int. Cl.⁴ : **C 07 D239/48**

(21) Anmeldenummer : **82104150.6**

(22) Anmeldetag : **12.05.82**

(54) **Verfahren zur Herstellung eines substituierten Pyrimidins.**

(30) Priorität : **15.05.81 CH 3190/81**

(43) Veröffentlichungstag der Anmeldung : **01.12.82 Patentblatt 82/48**

(45) Bekanntmachung des Hinweises auf die Patenterteilung : **02.01.86 Patentblatt 86/01**

(84) Benannte Vertragsstaaten : **AT DE FR GB IT SE**

(56) Entgegenhaltungen :
**DE-A- 2 010 166**
**DE-A- 2 313 361**
**DE-A- 2 559 747**
**JOURNAL OF MEDICINAL CHEMISTRY, Band 24, Nr. 8, August 1981, Seiten 933-941, Washington (USA); B.ROTH et al.: "2,4-Diamino-5-benzylpyrimidines and analogues as antibacterial agents. 5. 3',5'-Dimethoxy-4'-substituted-benzyl analogues of trimethoprim"**
**Die Akte enthält technische Angaben, die nach dem Eingang der Anmeldung eingereicht wurden und die nicht in dieser Patentschrift enthalten sind.**

(73) Patentinhaber : **SIEGFRIED AKTIENGESELLSCHAFT**
**CH-4800 Zofingen (CH)**

(72) Erfinder : **Saner, Hans, Dr.**
**Untere Gasse**
**CH-4625 Oberbuchsiten (CH)**
Erfinder : **Gnehm, René, Dr.**
**Bornweg 8**
**CH-4665 Küngoldingen (CH)**

(74) Vertreter : **Jaeger, Klaus, Dr. et al**
**JAEGER & PARTNER Patentanwälte Bergstrasse 48 1/2**
**D-8035 München-Gauting (DE)**

**EP 0 065 705 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung eines substituierten Pyrimidins, nämlich des 2,4-Diamino-5-[3,5-dimethoxy-4-(2-methoxyethoxy)-benzyl]-pyrimidins, das unter der freien Kurzbezeichnung Tetroxoprim ebenso wie das unter der freien Kurzbezeichnung Trimethoprim schon länger bekannte 2,4-Diamino-5-(3,4,5-trimethoxybenzyl)-pyrimidin als Chemotherapeuticum Bedeutung erlangt hat, sich gegenüber dem letzteren aber insbesondere durch seine Wasserlöslichkeit auszeichnet.

Es sind bis lang vier Wege zur Herstellung von Tetroxoprim bekanntgeworden :

Einer geht vom 3,5-Dimethoxy-4-(2-methoxyethoxy)-benzaldehyd aus, den man durch Reaktion mit 3-Alkoxypropionitril in Gegenwart eines basischen Katalysators in das entsprechende β-Alkoxy-α-[3,5-dimethoxy-(2-methoxyethoxy)-benzyl]-acrylnitril überführt ; letzteres wird dann durch die zum Pyrimidin-Ringschluss führende Umsetzung mit Guanidin in das Tetroxoprim umgewandelt.

Ein zweites Verfahren unterscheidet sich vom vorgenannten dadurch, dass 3-(Imidazolyl-1)-propionitril anstelle von 3-Methoxypropionitril eingesetzt wird.

Nach einem dritten bekanntgewordenen Verfahren wird 3,5-Dimethoxy-4-(2-methoxyethoxy)-hydrozimtsäure-ethylester durch Reaktion mit Guanidin in Gegenwart von Natriummethylat zum 2-Amino-4-hydroxy-5-[3,5-dimethoxy-4-(2-methoxyethoxy)-benzyl]-pyrimidin umgesetzt, dessen OH-Gruppe anschliessend mittels Phosphoroxychlorid durch Chlor ersetzt wird. Aus dem 5-Chlor-Derivat erhält man dann durch Aminolyse bei 160 °C das Tetroxoprim.

Nach dem vierten bekannten Verfahren geht man von 2,4-Diamino-5-(3,5-dimethoxy-4-hydroxybenzyl)-pyrimidin aus und verethert dessen OH-Gruppe durch Reaktion mit p-Toluolsulfonsäure-2-methoxyethylester in Gegenwart eines basischen Katalysators.

Für die Herstellung des Trimethoprims, dessen Struktur derjenigen des Tetroxoprims weitestgehend analog ist, sind dagegen auffällig zahlreiche Verfahren bekanntgeworden. Ein Grossteil derselben führt über ein Acrylnitril-Zwischenprodukt, das durch Reaktion von Trimethoxybenzaldehyd mit einem in β-Stellung mono- oder disubstituierten Propionitril erhalten wird und mit dem sich dann durch Umsetzung mit Guanidin der Pyrimidinring aufbauen lässt. Dieser an sich schon früh bekanntgewordene Weg präsentiert sich unter dem Gesichtspunkt der Zugänglichkeit der Ausgangsstoffe als der interessanteste ; er ist jedoch mit dem Handicap verbunden, dass die Reaktion zwischen Benzaldehyd und Propionitril in der Regel Isomerengemische, insbesondere Gemische aus einem Benzyl- und einem Benzalisomeren

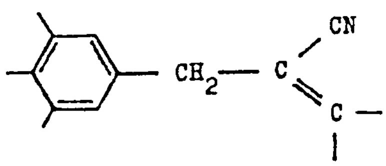 bzw. 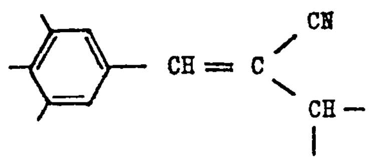

sowie auch polymere Nebenprodukte entstehen lässt, was naturgemäss zu Schwierigkeiten bei der Isolierung des Produktes und zu empfindlichen Ausbeuteschmälerungen führt. Ursachen und Auswirkungen von Gleichgewichtsverschiebungen zwischen den genannten zwei Isomeren scheinen im einzelnen noch nicht bis ins letzte geklärt zu sein ; gemäss Angaben der Literatur wird die Entstehung solcher Isomere nicht nur durch die Art der β-Substitution des Ausgangs-Propionitrils, sondern auch durch die Ringsubstituenten des Benzaldehyds, namentlich denjenigen in para-Stellung massgeblich beeinflusst. Nach vorherrschender Meinung lassen sich die bei der Trimethoprim-Herstellung dadurch entstehenden Schwierigkeiten am elegantesten umgehen, indem man von Propionitrilen ausgeht, die in β-Stellung mit einer Aminogruppe, insbesondere mit einer Morpholino- oder Anilinogruppe substituiert sind.

Umso überraschender muss es erscheinen, dass, soweit aus der zur Veröffentlichung gelangten Patentliteratur erkennbar wird, von der vorstehend erwähnten Verfeinerung des Benzaldehyd-/Propionitril-Verfahrens, die sich bei der Trimethoprim-Synthese bewährt zu haben scheint, im Falle der Tetroxoprim-Synthese kein erfolgreicher Gebrauch gemacht werden konnte. Das unter den eingangs aufgezählten, den Stand der Technik repräsentierenden Tetroxoprim-Herstellungsverfahren an zweiter Stelle genannte Vorgehen greift zwar den Gedanken der Verwendung von β-aminosubstituierten Propionitrilen auf ; der in der betreffenden Veröffentlichung (DE-OS-2 617 967) für das erhaltene Tetroxoprim angegebene Schmelzpunkt von 153 °C lässt jedoch nicht auf hohe Reinheit schliessen, und eigene Versuche zur Nacharbeit jenes Verfahrens haben fragwürdige Resultate geliefert.

Es hat sich nun gezeigt, dass die Vorteile der Verwendung eines in β-Stellung aminosubstituierten Propionitrils bei der Tetroxoprim-Herstellung dann zu auffallenden Vorteilen bezüglich Ausbeute und Reinheit des Endproduktes führen, wenn man beim Pyrimidin-Ringschluss ausgeht von einem in β-Stellung mit Anilin substituierten Acrylnitril-Zwischenprodukt.

Die Erfindung besteht demgemäss in einem Verfahren zur Herstellung von 2,4-Diamino-5-[3,5-dimethoxy-(2-methoxyethoxy)-benzyl]-pyrimidin der Formel I

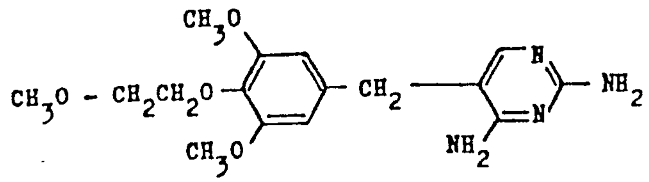

(I)

welches dadurch gekennzeichnet ist, dass man β-Anilino-α-[3,5-dimethoxy-(2-methoxyethoxy)-benzyl]-acrylnitril der Formel II

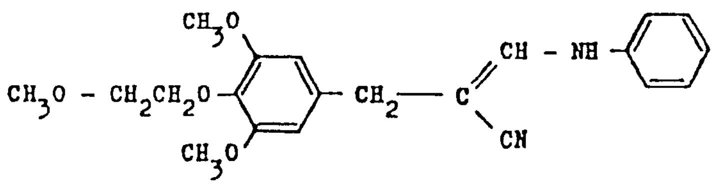

(II)

mit der freien Guanidinbase in äthanolischer Lösung unter Rückfluß zur Reaktion bringt.

Das als Ausgangsmaterial benützte, bisher nicht beschriebene β-Anilino-α-[3,5-dimethoxy-4-(2-methoxyethoxy)-benzyl]-acrylnitril ist leicht zugänglich bei analoger Anwendung der Methoden, die in CH-PS 574 399 für die Herstellung von β-Anilino-α-3,4,5-trimethoxybenzylacrylnitril beschrieben worden sind. Nach Umkristallisieren aus Toluol zeigt es einen Schmelzpunkt von 106 °C.

Eine zweckmässige Art der Durchführung des Verfahrens der Erfindung wird nachstehend anhand eines Beispiels im Detail beschrieben.

Beispiel

Man löst 115 g (5 mol) Natriummetall in 1 725 ml absolutem Ethanol, gibt 200 g (2 mol) Guanidinhydrochlorid (95 %) zu, erhitzt während 1 Stunde auf Rückfluss-Temperatur und filtriert nach dem Erkalten die Lösung der freigesetzten Guanidinbase vom abgeschiedenen Kochsalz ab. Der Lösung werden anschliessend 368 g (1 mol) β-Anilino-α-[3,5-dimethoxy-4-(2-methoxyethoxy)-benzyl]-acrylnitril zugegeben. Nach 3 Stunden Erhitzen unter Rückfluss wird das Volumen des Reaktionsgemisches durch Abdestillieren von Ethanol auf ca. ein Drittel reduziert, bis sich kristallines Tetroxoprim abzuscheiden beginnt. Nach Abkühlen auf 0 bis 5 °C, Filtrieren, Nachwaschen mit wenig Aceton und Trocknen bei 60 °C erhält man 314 g (94 % der Theorie) Tetroxoprim vom Smp. 158-161 °C.

**Patentanspruch**

Verfahren zur Herstellung von 2,4-Diamino-5-[3,5-dimethoxy-4-(2-methoxyethoxy)-benzyl]-pyrimidin der Formel I

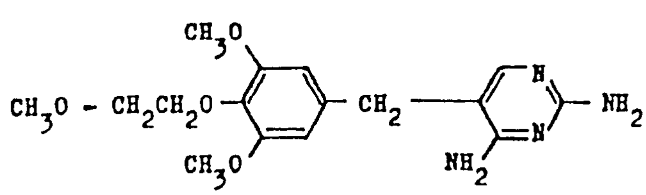

(I)

dadurch gekennzeichnet, dass man β-Anilino-α-[3,5-dimethoxy-4-(2-methoxyethoxy)-benzyl]-acrylnitril der Formel II

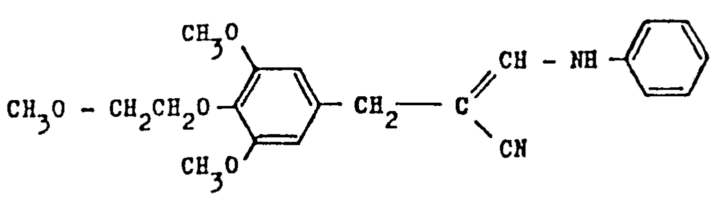

(II)

mit der freien Guanidinbase in äthanolischer Lösung unter Rückfluß zur Reaktion bringt.

**Claim**

A process for the preparation of 2,4-diamino-5-(3,5-dimethoxy-4-(2-methoxyethoxy)-benzyl)-pyrimidine represented by formula I

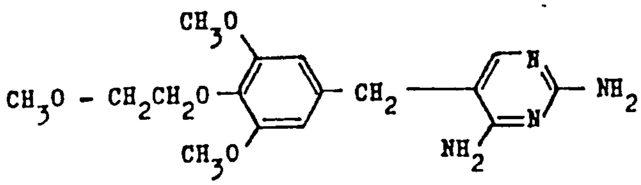
(I)

characterized in that β-anilino-α-(3,5-dimethoxy-4-(2-methoxyethyl)-benzyl)-acrylonitril represented by formula II

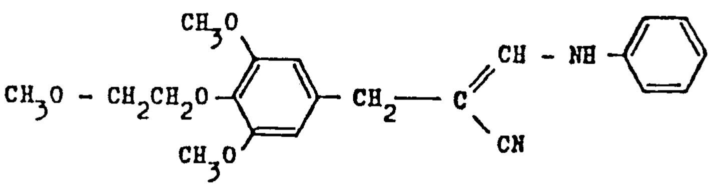
(II)

is brought to reaction with guanidine as a free base in a solution containing ethanol under reflux.

**Revendication**

Procédé de préparation de la diamino-2,4 [diméthoxy-3,5 (méthoxy-2 éthoxy)-4 benzyl]-5 pyrimidine, corps qui répond à la formule I

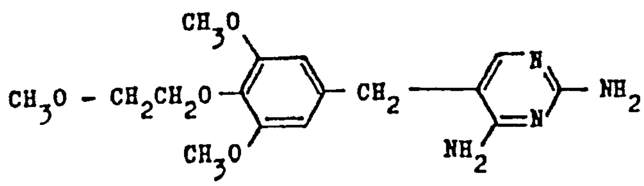
(I)

procédé caractérisé en ce qu'on fait réagir à reflux le β-anilino-α-[diméthoxy-3,4 (méthoxy-2 éthoxy)-4 benzyl]-acrylonitrile, corps qui répond à la formule II

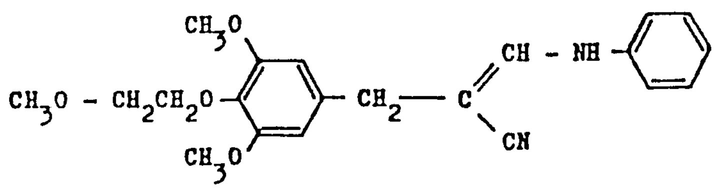
(II)

avec la guanidine basique libre, en solution éthanolique.